# EUROPEAN PATENT SPECIFICATION

(11) **EP 1 468 719 B1**
(45) Date of publication and mention of the grant of the patent: **26.04.2006**
(21) Application number: 03029945.7
(22) Date of filing: 29.12.2003
(51) Int. Cl.: B01D 53/22, B01D 69/12, B01D 71/26, B01D 71/70, C07C 7/144, C10G 31/11, B01D 69/14, B01D 71/82

(54) **Use of a composite membrane for the separation of hydrocarbons comprising a transition metal salt-polymer mixture separation layer**
Verwendung einer Kompositmembran zur Trennung von Kohlenwasserstoffen mit einer Übergangsmetall-Polymer-Trennschicht
Application d'une membrane composite pour la séparation d'hydrocarbures avec une couche de séparation comprenant un mélange polymère-métaux de transition

(30) Priority: 11.04.2003 KR 2003022842
(43) Date of publication of application: 20.10.2004
(73) Proprietor: Korea Institute of Science and Technology, Seoul 136-791 (KR)
(72) Inventor: Kim, Jong Hak, Seoul 132-741 (KR); Kang, Yong Soo, Seongbuk-gu Seoul 136-784 (KR); Jung, Bumsuk, Seoul 136-791 (KR); Won, Jongok, Hansin Apt. 103-602, Seoul 130-775 (KR); Min, Byoung Ryul, Seoul 120-160 (KR); Kim, Hoon Sik, Daewoo Apt. 106-1305, Seoul 136-767 (KR)
(74) Representative: Diehl & Partner

(56) References cited:
- EP-A- 0 634 204
- US-A- 5 015 268
- US-A- 5 670 051
- US-A1- 2001 013 273
- US-A1- 2001 015 334

## Description

The present invention relates to the use of a facilitated transport membrane with an improved permeance and selectivity to alkene hydrocarbons. In particular, the present invention relates to a facilitated transport membrane prepared by forming a solid transition metal salt-polymer membrane consisting of a transition metal salt and a rubbery polymer capable of dispersing the transition metal salt on the molecular scale; and coating the solid membrane on a porous supported membrane with good permeance and superior mechanical strength. The facilitated transport membrane is characterized in that its permeance and selectivity to alkene hydrocarbons is high and in that the transition metal ion in the transition metal salt-polymer membrane maintains its activity as a carrier for alkene hydrocarbons even under long-term dry operating conditions.

Alkene-series hydrocarbons, such as ethylene and propylene, are important raw materials that form the basis of the current petrochemical industry. Alkene hydrocarbons are primarily produced by pyrolysis of naphtha obtained from a petroleum refining process. They are important raw materials that form the basis of the current petrochemical industry. However, they are generally produced along with alkane hydrocarbons such as ethane and propane. Thus, alkene hydrocarbons/alkane hydrocarbons separation technology is of significant importance in the related industry.

Currently, the traditional distillation process is used mostly for the separation of alkene/alkane mixtures such as ethylene/ethane or propylene/propane. The separation of such mixtures, however, requires the investment of large-scale equipment and high-energy cost due to their similarity in molecular size and physical properties such as relative volatility.

In the distillation process used hitherto, for example, a distillation column having about 120-160 trays should be operated at a temperature of -30°C and a high pressure of about 20 atm for separation of an ethylene and ethane mixture. For separation of a propylene and propane mixture, a distillation column having about 180-200 trays should be operated at a temperature of -30°C and a pressure of about several atms in the reflux ratio of 10 or more. As such, there has been a continuous need for the development of a new separation process that can replace the prior distillation process, because the separation process requires the investment of large-scale equipment and high-energy cost.

A separation process that could be considered as a replacement for said prior distillation process is one that uses a separation membrane. Separation membrane technology has progressed remarkably over the past few decades in the field of separating gas mixtures, for example, the separation of nitrogen/oxygen, nitrogen/carbon dioxide and nitrogen/methane, etc.

However, the satisfactory separation of alkene/alkane mixtures cannot be accomplished by using traditional gas separation membranes because alkene and alkane are very similar in terms of their molecular size and physical properties. A facilitated transport membrane based on a different concept from the traditional gas separation membranes is considered to be an alternative process for separation membrane that can achieve excellent separation performance for alkene/alkane mixtures.

The separation of mixtures in a separation process using a separation membrane is achieved by the difference in permeance between the individual components constituting the mixtures. Most materials of a separation membrane have many limitations on their application because of an inverse correlation between permeance and selectivity. However, the concurrent increase of permeance and selectivity is made possible by applying a facilitated transport phenomenon. Consequently, the scope of their application can be considerably increased. If a carrier capable of selectively and reversibly reacting with a specific component of a mixture is present in a separation membrane, mass transport is facilitated by additional material transport generated from a reversible reaction of a carrier and a specific component. Therefore, overall mass transport can be indicated by Fick's law and the sum of material transport caused by a carrier. This phenomenon is referred to as facilitated transport.

A supported liquid membrane is an example of a membrane prepared by applying the concept of facilitated transport. The supported liquid membrane is typically prepared by filling a porous thin layer with a solution that is obtained by dissolving a carrier capable of facilitating mass transport in a solvent such as water, etc. Such a supported liquid membrane has succeeded to a certain extent.

Steigelmann and Hughes, for example, prepare a supported liquid membrane in which the selectivity of ethylene/ethane is about 400-700 and the permeance of ethylene is 60 GPU [1 GPU = 1 x 10⁻⁶ cm³ (STP)/cm²·sec·cmHg], which are satisfactory performance results for permeance separation (see U.S. Pat. Nos. 3,758,603 and 3,758,605). However, the supported liquid membrane exhibits the facilitated transport phenomenon only under wet conditions. There is the inherent problem in that the initial permeance separation performance cannot be maintained for an extended time due to solvent loss and the decrease of separation performance with time..

In order to solve the problem of the supported liquid membrane, Kimura, etc., suggests a method that enables facilitated transport by substituting a suitable ion in an ion-exchange resin (see U.S. Pat. No. 4,318,714). This ion-exchange resin membrane also has a drawback, however, in that the facilitated transport phenomenon is exhibited only under wet conditions, similar to the supported liquid membrane.

Ho suggests another method for the preparation of a complex by using water-soluble glassy polymer such as polyvinyl alcohol (see U.S. Pat. Nos. 5,015,268 and 5,062,866). However, the method also has a drawback in that satisfactory results are obtained only when feed gas is saturated with water vapor by passing the feed gas through water or when a membrane is swelled with ethylene glycol or water.

In all the instances described above, the separation membrane must be maintained in wet conditions that enable the membrane to contain water or other similar solvents. When a dry hydrocarbon gas mixture - for example, an alkene/alkane mixture free of a solvent such as water - is separated by using the membrane, solvent loss is unavoidable with time. Therefore, a method is necessary for periodically feeding a solvent to a separation membrane in order to continuously sustain the wet condition of the separation membrane. It is, however, rarely possible for the method to be applied to a practical process because the membrane is not stable.

Kraus, etc., develops a facilitated transport membrane by using another method (see U.S. Pat. No. 4,614,524). According to the patent, a transition metal is substituted in an ion-exchange membrane such as Nafion, and the membrane is plasticized with glycerol, etc. The membrane could not be utilized, however, in that its selectivity is as low as about 10 when dry feed is used. The membrane also has no selectivity when a plasticizer is not used. Furthermore, a plasticizer is lost with time.

In view that a usual polymer separation membrane cannot separate alkene/alkane mixtures having similar molecular size and physical properties, as described above, use of a facilitated transport membrane capable of selectively separating only alkane is necessary. In conventional facilitated transport membranes, however, the activity of a carrier is maintained by using the following method: filling a solution containing a carrier into the porous membrane, adding a volatile plasticizer, or saturating a feed gas with water vapor. Such a membrane cannot be utilized due to the problem of declining stability of the membrane since components constituting the membrane are lost with time. There is also the problem of later having to remove solvents such as water, etc., which are periodically added in order to sustain activity, from the separated product.

Therefore, there is a need for the application of a separate membrane that can replace the prior distillation process requiring the investment of large-scale equipment and high-energy cost in the separation of alkene/alkane mixtures, in which the separation membrane does not contain volatile components and has high selectivity and permeance so that it can maintain the activity even under long-term dry operating conditions.

In order to solve the above-mentioned problems, the present inventors used a facilitated transport membrane using a polymer electrolyte prior to the present invention (see Korean Pat. Nos. 315894 and 315896, Korean Pat. Appl. No. 2001-8793).
EP-A-0634204 discloses the use of a supported polydimetylsiloxane membrane for separating alkene hydrocarbons from a hydrocarbon mixture under wet conditions, i.e. a liquid carrier fluid comprising AgNO₃ is provided on the permeate side of the membrane.
US-A-20010013273 discloses the use of facilitated transport membranes for separating alkene hydrocarbons from a hydrocarbon mixture wherein the membrane comprises a hydrophilic polymer, preferably poly(2-ethyl-2-oxazoline) or polyvinylpyrrolidone.

In view of foregoing, it is a primary object of the present invention to use a facilitated transport membrane for separating alkehydrocarbons from hydrocarbon mixtures, which is prepared by introducing a solid transition metal salt-polymer membrane into a facilitated transport membrane, in which the facilitated transport membrane has a high permeance and selectivity to unsaturated hydrocarbons such as alkene even under dry conditions and has no problems in stability, such as carrier loss, to be able to sustain the activity for a prolonged period of time.

That facilitated transport membrane has its prominent characteristics in separating alkene hydrocarbons from mixtures of alkene hydrocarbons and alkane hydrocarbons by coating a solid transition metal salt-polymer membrane consisting of a transition metal salt and a polymer having no functional group capable of forming a complex with the transition metal on a porous supported membrane and being selected from polyolefines, polysiloxanes and mixtures and copolymers thereof. The facilitated transport membrane has a high permeance and selectivity to alkene and maintains the activity even under long-term dry operating conditions without feed of liquid solvents.

The foregoing and other objects and features of the present invention will become more fully apparent from the following description and appended claims.

A facilitated transport membrane is prepared by coating a transition metal-polymer membrane consisting of a transition metal salt and a polymer on a porous supported membrane, in which the polymer has no functional group capable of forming a complex with the transition metal salt and does not dissolve but can physically disperse the transition metal salt. In the facilitated transport membrane according to the present invention, the transition metal salt is uniformly dispersed in the polymer matrix on the molecular scale. The double bonds of alkenes selectively and reversibly react with the ion of transition metal in the facilitated transport membrane to facilitate the transport of alkenes. Consequently, the facilitated transport membrane can selectively separate alkenes.

The facilitated transport membrane according to the present invention is based on a different concept from a prior facilitated transport membrane using transition metal salt-polymer electrolyte layer disclosed in Korean Pat. Nos. 315894 and 315896 and Korean Pat. Appl. No. 2001-8793, in that the facilitated transport membrane according to the present invention uses a transition metal salt-polymer membrane comprising a polymer that does not form a complex with a transition metal salt.

The present invention is described in detail below.

The facilitated transport membrane used according to the present invention comprises a transition metal salt-polymer membrane and a porous supported membrane supporting the transition metal salt-polymer membrane, in which the polymer constituting the transition metal salt-polymer membrane has no functional group capable of forming a complex with the transition metal salt and does not dissolve but can physically disperse the transition metal salt.

Hydrocarbon mixtures to be separated in the present invention contain at least one alkene hydrocarbon and/or at least one alkane hydrocarbon and/or other gas. The alkene hydrocarbon includes ethylene, propylene, butylene, 1,3-butadiene, isobutylene, isoprene, and others; the alkane hydrocarbon includes methane, ethane, propane, butane, isobutane, pentane isomers, and others; and other gas includes oxygen, nitrogen, carbon dioxide, carbon monoxide, water, and others.

Any porous support membranes having good permeance and sufficient mechanical strength may be used in the present invention. For example, a conventional porous polymer membrane, a ceramic membrane or any other proper membranes may be used. Plate, tubular, hollow or other shapes of supported membranes may also be used in the invention.

A transition metal salt acting as a carrier and a polymer capable of dispersing the transition metal salt uniformly on the molecular scale have a substantial effect on the selective separation of alkene hydrocarbon. The properties of the transition metal salt and polymer determine the selective permeation separation of alkene hydrocarbon from the corresponding alkane hydrocarbon.

A transition metal salt is uniformly dispersed as a form of ion aggregate in a polymer matrix immediately after a facilitated transport membrane is prepared. When an alkene hydrocarbon is introduced into the membrane, the alkene hydrocarbon forms a complex with the transition metal salt. Consequently, the ion aggregate is dissociated into a free ion to directly participate in the facilitated transport of an alkene hydrocarbon (see J. H. Kim, B. R. Min, J. Won, Y. S. Kang, Chem. Eur. J., 2002, 8, 650). That is, a cation of a transition metal in the membrane interacts with an anion of salt, a polymer and an alkene hydrocarbon. Therefore, they must be properly selected to obtain a separation membrane having high selectivity and permeance.

It is well known that a transition metal reacts reversibly with alkene hydrocarbon in a solution (see J. P. C. M. Van Dongen, C. D. M. Beverwijk, J. Organometallic Chem., 1973, 51, C36). The ability of a transition metal ion as a carrier is determined by the size of the π -complexation formed with alkene, which is determined by electronegativity. Electronegativity is a measure of the relative strength of an atom to attract covalent electrons when the atom is bonded with other atoms. The electronegativity values of transition metals are shown in Table 1 below:

**Table 1 Electronegativity Values of Transition Metals**

| Transition metal. | Sc | V | Cr | Fe | Ni | Cu |
|---|---|---|---|---|---|---|
| Electronegativity | 1.4 | 1.6 | 1.7 | 1.8 | 1.9 | 1.9 |
| Transition metal | Y | Nb | Mo | Ru | Pd | Ag |
| Electronegativity | 1.3 | 1.6 | 2.2 | 2.2 | 2.2 | 1.9 |
| Transition metal | La | Ta | W | Os | Pt | Au |
| Electronegativity | 1.0 | 1.5 | 2.4 | 2.2 | 2.3 | 2.5 |

If the electronegativity of a metal is high, the metal atom will more strongly attract electrons when it is bonded with other atoms. If the electronegativity of a metal is too high, the metal is not suitable as a carrier of the facilitated transport due to increased possibility of the irreversible reaction of the metal and π -electrons of alkene. On the other hand, if the electronegativity of a metal is too low, the metal cannot act as a carrier because of its low interaction with alkene.

Therefore, the electronegativity of a metal is preferably in the range of from 1.6 to 2.3 so that the transition metal ion reacts reversibly with alkene. Preferred transition metals within the above ranges include Mn, Fe, Co, Ni, Cu, Mo, Tc, Ru, Rh, Pd, Ag, Re, Os, Ir, Pt, or isomers and complexes thereof.

An anion of a transition metal salt has an important role in improving the reversible reactivity of a metal transition ion and alkene hydrocarbons, particularly in improving the reverse reaction rate, allowing readily separation of alkenes that form a complex with a transition metal in effluent. Therefore, it is preferable to select an anion of a transition metal salt that has low lattice energy in respect of a given cation of a transition metal, in order to readily solvate a transition metal salt and improve solvation stability in the facilitated transport membrane used according to the present invention. The lattice energy of representative transition metal salts is given in Table 2 below.

**Table 2 Lattice Energy of Metal Salts [kJ/mol]^{a)}**

| | Li⁺ | Na⁺ | K⁺ | Ag⁺ | Cu⁺ | Co²⁺ | Mo²⁺ | Pd²⁺ | Ni²⁺ | Ru³⁺ |
|---|---|---|---|---|---|---|---|---|---|---|
| F | 1036 | 923 | 823 | 967 | 1060^{b)} | 3018 | | | 3066 | |
| Cl⁻ | 853 | 786 | 715 | 915 | 996 | 2691 | 2733 | 2778 | 2772 | 5245 |
| Br⁻ | 807 | 747 | 682 | 904 | 979 | 2629 | 2742 | 2741 | 2709 | 5223 |
| I⁻ | 757 | 704 | 649 | 889 | 966 | 2545 | 2630 | 2748 | 2623 | 5222 |
| CN⁻ | 849 | 739 | 669 | 914 | 1035 | | | | | |
| NO₃⁻ | 848 | 756 | 687 | 822 | 854 ^{b)} | 2626 | | | 2709 | |
| BF₄⁻ | 705 ^{b)} | 619 | 631 | 658 ^{b)} | 695 ^{b)} | 2127 | | | 2136 | |
| ClO₄⁻ | 723 | 648 | 602 | 667 ^{b)} | 712 ^{b)} | | | | | |
| CF₃SO₃⁻ | 779 ^{b)} | 685 ^{b)} | 600 ^{b)} | 719 ^{b)} | 793 ^{b)} | | | | | |
| CF₃CO₂⁻ | 822 ^{b)} | 726 ^{b)} | 658 ^{b)} | 782 ^{b)} | 848 ^{b)} | | | | | |
| ^{a)} See H.D.B. Jenkins, CRC Handbook, 74^{th} Ed., 12-13 (1993) | | | | | | | | | | |
| ^{b)} Complexation energy for the formation of an ion pair such as M⁺_{(g)} + X⁻_{(g)} ⇒ MX_{(g)} is calculated by using the Becke3LYP method (Becke3/6-311 + G*//Becke3/6-311 + G*) of Density Function Theory (DFT), which uses a basic set function of 6-311+G*. The calculated value linear-regresses with the lattice energy described in literature a). It is confirmed that there is good linearity with a correlation coefficient of at least 0.94. Thus, the lattice energy of salts that are not described in the literature is estimated by using the correlation obtained above. | | | | | | | | | | |

An anion constituting a transition metal salt of the facilitated transport membrane used according to the present invention is preferably selected from anions having a lattice energy of 2500 kJ/mol or less in order to suppress the tendency to form a strong ion pair with a cation and to improve solvation stability. Among the metal salts listed in Table 2, the anions may include F⁻, Cl⁻, Br⁻, I⁻, CN⁻, NO₃⁻, and BF₄⁻ , which constitute salts with Ag⁺ or Cu⁺. Anions applicable to the present invention, however, are not limited only to those listed in Table 2.

The solution stability of anions is generally exhibited in the order of F⁻<< Cl⁻< Br⁻<I⁻~ SCN⁻< ClO₄⁻~ CF₃SO⁻₃< BF₄⁻~ AsF6⁻, in which lattice energy decreases, i.e., the tendency of the anions to form strong ion pairs with cations of metal salts is reduced as it progresses toward the right. These various anions, which are desirable for use in the facilitated transport membrane used according to the present invention due to low lattice energy, have been widely utilized in electrochemical devices such as batteries or electrochemical capacitors, etc. Such anions may include SCN⁻, ClO₄⁻, CF₃SO₃⁻, BF₄⁻, AsF₆⁻, PF₆⁻, SbF₆⁻, AlCl₄⁻, N(SO₂CF₃)₂⁻, C(SO₂CF₃)₃⁻, and others, but various anions in addition to those illustrated herein.may be used in the present invention. Anions coinciding with the object of the present invention are not limited to those described herein.

Further, monosalts as well as complex salts of transition metals, such as (M₁)ₓ(M₂) _{x'}Y₂, (M₁)ₓ(X₁)_{y}(M₂)_{x'}(X₂)_{y'} (wherein M₁ and M₂ represent a cation; X, X₁ and X₂ represent an anion; and x, x', y and y' represent an atomic value) or organic salt-transition metal salts, or physical mixtures of at least one salt may be used in the facilitated transport separation of the present invention.

Some examples of the complex salts of transition metals may include RbAg₄I₅, Ag₂HgI₄, RbAg₄I₄CN, AgHgSI, AgHgTeI, Ag₃SI, Ag₆I₄WO₄, Ag₇I₄AsO₄, Ag₇I₄PO₄, Ag₁₉I₁₅P₂O₇, Rb₄Cu₁₆I₇Cl₁₃, Rb₃Cu₇Cl₁₀, AgI-(tetraalkyl ammonium iodide), AgI-(CH₃)₃SI, C₆H₁₂N₄·CH₃I-CuI, C₆H₁₂N₄·4CH₃Br-CuBr, C₆H₁₂N₄·4C₂H₅Br-CuBr, C₆H₁₂N₄·4HCl-CuCl, C₆H₁₂N₂·2CH₃I-CuI, C₆H₁₂N₂·2CH₃Br-CuBr, C₆H₁₂N₂·2CH₃Cl-CuCl, C₅H₁₁NCH₃I-CuI, C₅H₁₁NCH₃Br-CuBr, C₄H₉ON· CH₃I-CuI, and others. However, numerous combinations similar to these complex salts or mixtures of salts can be used within the spirit of the present invention. As such, the present invention is not limited to those illustrated above.

The polymer used in the present invention must have no functional group containing oxygen and/or nitrogen, which reduces a transition metal ion to a transition metal particle. Also, the polymer must have no functional group capable of forming a complex with a transition metal salt, and so disperse a transition metal salt in a polymer matrix on the molecular scale. In the present invention, the polymer is selected from the group consisting of polyolefins, polysiloxanes, copolymers and mixtures thereof. Some examples of representative polymers include, but not limited to, polyethylene, polypropylene, polyethylene-co-propylene copolymer, polydimethyl siloxane, and copolymers and blends thereof.

The facilitated transport membrane used according to the present invention can preferably be prepared by the following two methods.

One of the methods is a conventional method comprising the steps of dissolving a polymer and a transition metal salt in a liquid solvent to obtain a coating solution; coating the coating solution on a porous supported membrane; and drying the resultant product. Any liquid solvent that dissolves a polymer and a transition metal but does not impair a porous supported membrane can use in the method.

The other method is used when a uniform solution cannot be obtained by dissolving a polymer and a transition metal in a liquid solvent. The method comprises the steps of first dissolving a polymer in a solvent to obtain a solution; coating the solution on a porous supported membrane; completely evaporating the solvent to form a polymer membrane; and coating a solution containing a transition metal salt on the polymer membrane. In the method, the solution containing a transition metal salt must not dissolve the polymer membrane.

The facilitated transport membrane used according to the present invention exhibits substantially high selectivity to alkene hydrocarbons, which is superior to prior selectivity to alkene hydrocarbons. Since the solid polymer matrix in the facilitated transport membrane has no functional group capable of forming a transition metal salt, the transition metal salt can be uniformly dispersed in the polymer matrix.
Furthermore, the present invention does eliminate known problems, such as reduction of a transition metal ion to a transition metal particle, in using a polymer matrix having a functional group containing oxygen and/or nitrogen. Of special interest is that the facilitated transport membrane exhibits low separation performance in early stage of the permeance test, but shows that both permeance and selectivity are increased over the permeance test, but shows that both permeance and selectivity are increased over time. The phenomenon is because the transition metal salt is simply dispersed as a form of ion aggregate in the polymer matrix in early stage and is dissociated into a free ion of transition metal by an alkene hydrocarbon with time. Consequently, the free ion acts as a carrier for an alkene hydrocarbon to facilitate the transport of an alkene hydrocarbon.

The examples below illustrate the present invention in detail, but the invention is not limited to the scope thereof.

### EXAMPLE 1

0.2g of polydimethyl siloxane (PDMS, RTV, 3-1744, Dow Corning, M_{w} = 48,000) was dissolved in 0.8g of 1-hexene to obtain a uniform and clear polymer solution (polymer concentration = 20 wt%). The procedure was repeated to obtain four solutions.

Then, 0.264g of silver tetrafluoroborate (AgBF₄, 98%, Aldrich Co.), 0.280g of silver perchlorate (AgClO₄, 99.9%, Aldrich Co.), 0.347g of silver trifluoromethane sulfonate (AgCF₃SO₃ or AgTf, 99+%, Aldrich Co.) or 0.465g of silver hexafluoroantimonate (AgSbF₆, 98%, Aldrich Co.) were added to the respective four solutions to obtain four solutions of polymer:silver ion = 2:1 in mole ratio. The resulting respective solutions were coated on respective four polyester porous membranes (track etched membrane, 0.1 µm polyester, Whatman) by using a Mayer bar. The respective thickness of substantial separation layers of determined by a high resolution electron microscope (SEM) was about 2 µm. The separation membranes thus prepared were completely dried in a dry oven for 2 hrs and a vacuum oven for 48 hrs at room temperature.
The pressure of the top portion was 40 psig and the pressure of the permeation portion was 0 psig. The permeance of a permeated gas was determined by using a soap-bubble flow meter, and the composition ratio was determined by the ratio of two peaks obtained from gas chromatography. Gas permeance was expressed in GPU [10⁻⁶ cm³(STP)/cm²· cmHg· sec]. The permeance and selectivity of pure PDMS membrane and PDMS/Ag salt membranes measured at their steady state (about 2hrs after the start of the separation performance test) are shown in Table 3 below.

**Table 3**

| | Permeance of propylene (GPU) | Permeance of propane (GPU) | Selectivity to propylene/propane |
|---|---|---|---|
| PDMS | 24.6 | 22.4 | 1.1 |
| PDMS/AgBF₄ | 12.9 | 0.08 | 158.1 |
| PDMS/AgClO₄ | 12.0 | 0.29 | 42.0 |
| PDMS/AgCF₃SO₃ | 7.7 | . 0.82 | 9.4 |
| PDMS/AgSbF₆ | 9.2 | 1.13 | 8.1 |

As seen in Table 3, pure PDMS membrane did not exhibit the separation performance, while all PDMS membranes containing silver salt exhibited superior selectivity in comparison with the pure PDMS membrane even though there was a difference in selectivity according to silver salt types.

### EXAMPLE 2

0.2g of polydimethyl siloxane (PDMS, RTV, 3-1744, Dow Corning, M_{w} = 48,000) was dissolved in 0.8g of 1-hexene to obtain a uniform and clear polymer solution (polymer concentration = 20 wt%). The procedure was repeated to obtain five solutions.

Then, 0.053g, 0.088g, 0.176g, 0.264g or 0.527g of silver tetrafluoroborate (AgBF₄, 98%, Aldrich Co.) was added to the respective five solutions to obtain five solutions of polymer: silver ion = 10:1, 6:1, 3:1, 2:1 or 1:1 in mole ratio. Thereafter, five PDMS/AgBF₄ membranes were prepared using the five solutions as described in Example 1 and their selectivity of a propylene/propane mixture (50:50vol%) over time was determined. The results are shown in Table 4 below.

**Table 4**

| Time | Mole ratio of PDMS:Ag ion | | | | |
|---|---|---|---|---|---|
| | 10:1 | 6:1 | 3:1 | 2:1 | 1:1 |
| 15min | 1.0 | 1.1 | 1.3 | 3.8 | 4.5 |
| 30min | 3.4 | 8.5 | 12.9 | 47.0 | 82.3 |
| 45min | 7.1 | 13.4 | 26.4 | 74.0 | 119.5 |
| 60min | 11.2 | 20.3 | 38.5 | 96.0 | 155.4 |
| 90min | 19.0 | 38.7 | 55.2 | 128.1 | 197.2 |
| 150mm | 18.4 | 53.4 | 80.9 | 150.4 | 214.6 |
| 210min | 19.2 | 53.0 | 84.6 | 155.4 | 203.5 |
| 270min | 22.1 | 58.1 | 84.2 | 160.2 | 210.7 |
| 330min | 20.4 | 56.0 | 83.5 | 157.5 | 208.4 |

As seen in Table 4, all membranes exhibited low selectivity in early stage and exhibited an increasing selectivity with time. This occurs because the silver salt is simply dispersed as a form of ion aggregate in the polymer matrix in early stage and is dissociated into a free silver ion by propylene gas with time. Consequently, the free ion acts as a carrier for propylene to facilitate the transport of propylene.

The time to approaching the steady state was about 150min at which the selectivity was rarely affected by the concentration of silver salt. Furthermore, the selectivity after the steady state was linearly increased with increasing silver salt concentration.

### EXAMPLE 3

0.2g of polydimethyl siloxane (PDMS, RTV, 3-1744, Dow Corning, M_{w} = 48,000) was dissolved in 0.8g of 1-hexene to obtain a uniform and clear polymer solution (polymer concentration = 20 wt%).

Then, 0.527g of silver tetrafluoroborate (AgBF₄, 98%, Aldrich Co.) was added to the solution to obtain polymer: silver ion = 1:1 in mole ratio. Thereafter, a PDMS/AgBF₄ membrane was prepared by using the method described in Example 1 and permeance of a propylene/propane mixture (50:50vol%) over time was determined. Table 5 below shows the permeance of propylene and propane.

**Table 5**

| Time | Permeance of propylene (GPU) | Permeance of propane (GPU) |
|---|---|---|
| 15min | 9.3 | 0.201 |
| 30min | 10.9 | 0.133 |
| 60min | 12.3 | 0.103 |
| 90min | 13.4 | 0.068 |
| 150min | 14.5 | 0.070 |
| 210min | 15.0 | 0.065 |
| 270min | 15.1 | 0.071 |
| 330min | 14.9 | 0.066 |

As seen in Table 5, the permeance of propylene was increased with time, while the permeance of propane was decreased with time. Consequently, the facilitated transport of propylene is improved with time. It shows that a silver salt disperses as a form of ion aggregate in a polymer matrix in early stage and is dissociated into a free ion of transition metal by propylene with time. Consequently, the free ion acts as a carrier for propylene to facilitate the transport of propylene.

### EXAMPLE 4

0.3g of polyethylene-co-propylene (EPR, M_{w} = 170,000, Aldrich Co.) was dissolved in 9.7g of tetrahydrofuran (THF) to obtain a uniform and clear polymer solution (polymer concentration = 3wt%). The polymer solution was coated on a porous polyester membrane (track etched membrane, 0.1 µm polyester, Whatman) using a Mayer bar. The membrane thus prepared was completely dried in a dry oven for 2hrs and a vacuum over for 24hrs at room temperature.

Thereafter, ethanol solution prepared by dissolving 0.287g of silver tetrafluoroborate (AgBF₄, 98%, Aldrich Co.) in O.5g of ethanol was coated on the membrane. The membrane thus prepared was completely dried in a dry oven for 2hrs and a vacuum oven for 48hrs at room temperature.

The membranes were evaluated on separation performance over time using using a propylene/propane mixture (50:50vol%) at room temperature. Table 6 below shows the permeance of propylene and propane.

**Table 6**

| Time | Permeance of propylene (GPU) | Permeance of propane (GPU) |
|---|---|---|
| 15mm | 2.2 | 1.91 |
| 30min | 2.3 | 1.64 |
| 45min | 2.5 | 0.69 |
| 60min | 3.3 | 0.24 |
| 90min | 5.7 | 0.14 |
| 120min | 6.8 | 0.13 |
| 180min | 6.9 | 0.13 |
| 240min | 7.0 | 0.13 |

As seen in Table 6, the permeance of propylene was increased with time, while the permeance of propane was decreased with time. It shows that a silver salt is simply dispersed as a form of ion aggregate in a polymer matrix in early stage and is dissociated into a free ion of transition metal by propylene gas with time.
Consequently, the free ion acts as a carrier for propylene to facilitate the transport of propylene.

### EXAMPLE 5

A PDMS/AgBF₄ membrane prepared in Example 3 was estimated on a long-term operation performance at room temperature. The separation performance was tested using a propylene/propane mixture (50:50vol%) under condition wherein the pressure of top portion was 40 psig and the pressure of permeation portion was 0 psig.

The permeance of a permeated gas was determined with a soap-bubble flow meter, and the composition ration was determined with gas chromatography to evaluate the long-term operation performance. Also, a poly(2-ethyl-2-oxazole) (POZ)/AgBF₄ membrane having a functional group containing oxygen, which is not according to the present invention, was evaluated on a long-term operation performance as described above. The results are presented in Table 7 below.

**Table 7**

| | PDMS/AgBF₄ | | POZ/AgBF₄ | |
|---|---|---|---|---|
| Time (hour) | Permeance of a gas mixture (GPU) | Selectivity of a gas mixture (propylene/propane) | Permeance of a gas mixture (GPU) | Selectivity of a gas mixture (propylene/propane) |
| 2 | 13.5 | 200.1 | 16 | 52 |
| 6 | 14.9 | 208.4 | 15 | 52 |
| 12 | 14.2 | 203.2 | 12 | 51 |
| 24 | 14.5 | 205.7 | 13 | 48 |
| 48 | 13.9 | 206.4 | 12 | 42 |
| 72 | 14.3 | 208.2 | 7 | 37 |
| 96 | 14.8 | 209.4 | 5 | 34 |
| 120 | 14.7 | 210.3 | 4 | 31 |
| 144 | 14.5 | 206.4 | 3 | 29 |

As Seen in Table 7 the permeance and selectivity of the POZ/AgBF₄ membrane continuously decreased with time, while the performance of the PDMS/AgBF₄ membrane rarely decreased and was maintained during a long-term operation of about 150hrs.

The facilitated transport membrane used according to the present invention exhibits substantially high selectivity to alkene hydrocarbons. Furthermore, the present invention does eliminates problems associated with the use of a polymer matrix having a functional group containing oxygen and/or nitrogen, such as reduction of a transition metal ion to a transition metal.

## Claims

1. Use of a facilitated transport membrane comprising a porous support membrane and a transition metal salt-polymer membrane consisting of a transition metal salt and a polymer selected from the group consisting of polyolefins, polysiloxanes, and copolymers and mixtures thereof, in which the transition metal salt is dispersed within the polymer which has no functional group capable of forming a complex with the transition metal salt,
for separating alkene hydrocarbons from hydrocarbon mixtures under dry operating conditions without feed of liquid solvents.

2. The use of the facilitated transport membrane according to Claim 1, wherein a cation of the transition metal salt has the electronegativity of 1.8~2.3.

3. The use of the facilitated transport membrane according to Claim 2, wherein the transition metal is one selected from the group consisting of Mn, Fe, Co, Ni, Cu, Mo, Tc, Ru, Rh, Pd, Ag, Re, Os, Ir, Pt, and isomers and complexes thereof.

4. The use of the facilitated transport membrane according to Claim 1, wherein the transition metal salt has a lattice energy of less than 2500 kJ/mol.

5. The use of the facilitated transport membrane according to Claim 4, wherein an anion of the transition metal salt is one selected from the group consisting of F⁻, Cl⁻, Br⁻, I⁻, CN⁻, NO₃⁻, SCN⁻, C10₄⁻, CF₃SO₃⁻, BF₄⁻, AsF₆⁻, PF₆⁻, SbF₆⁻, AlC1₄⁻, N (SO₂CF₃) ₂⁻ and C(SO₂CF₃)₃⁻.

6. The use of the facilitated transport membrane according to Claim 1, wherein the transition metal salt includes a complex salt of the transition metal or a mixture of the salts of the transition metal.

7. The use of the facilitated transport membrane according to Claim 1, wherein the polymer is one selected from the group consisting of polyethylene, polypropylene, polyethylene-co-propylene copolymer, polydimethyl siloxane, and copolymers and blends thereof.

8. The use of the facilitated transport membrane according to Claim 1, wherein the porous support membrane is a porous polymer membrane or ceramic membrane.

9. The use of the facilitated transport membrane according to Claim 1, wherein the hydrocarbon mixtures to be separated contain at least one alkene hydrocarbon and/or at least one alkane hydrocarbon and/or other gas.

10. The use of the facilitated transport membrane according to Claim 8 or 9, wherein the alkene hydrocarbon is one selected from the group consisting of ethylene, propylene, butylene, 1,3-butadiene, isobutylene, isoprene and mixtures thereof, the alkane hydrocarbon is one selected from the group consisting of methane, ethane, propane, butane, isobutane, pentane isomers and mixtures thereof, and other gas is one selected from the group consisting of oxygen, nitrogen, carbon dioxide, carbon monoxide, water and mixtures thereof.

## Patentansprüche

1. Verwendung einer Membran mit erleichtertem Stofftransport, die eine poröse Stützmembran sowie eine Übergangsmetallsalz-Polymermembran umfasst, die aus einem Übergangsmetallsalz und einem Polymer besteht, das aus der Gruppe gewählt ist, die aus Polyolefinen, Polysiloxanen und Kopolymeren und Mischungen davon besteht, wobei das Übergangsmetallsalz in dem Polymer, das keine Gruppe aufweist, die mit dem Übergangsmetallsalz einen Komplex bilden kann, dispergiert ist, zum Abtrennen von Alken-Kohlenwasserstoffen aus Kohlenwasserstoff-Mischungen unter trockenen Betriebsbedingungen, ohne Zugabe flüssiger Lösungsmittel.

2. Verwendung der Membran mit erleichtertem Stofftransport gemäss Anspruch 1, wobei das Kation des Übergangsmetallsalzes eine Elektronegativität von 1,8 - 2,3 hat.

3. Verwendung der Membran mit erleichtertem Stofftransport gemäss Anspruch 2, wobei das Übergangsmetall gewählt wird aus der Gruppe, die aus Mn, Fe, Co, Ni, Cu, Mo, Tc, Ru, Rh, Pd, Ag, Re, Os, Ir, Pt und deren Isomeren und Komplexen besteht.

4. Verwendung der Membran mit erleichtertem Stofftransport gemäss Anspruch 1, wobei das Übergangsmetallsalz eine Gitterenergie von weniger als 2500 kJ/mol aufweist.

5. Verwendung der Membran mit erleichtertem Stofftransport gemäss Anspruch 4, wobei ein Anion des Übergangsmetallsalzes gewählt wird aus der Gruppe, die aus F⁻, Cl⁻, Br⁻, I⁻, CN⁻, NO₃⁻, SCN⁻, ClO₄⁻, CF₃SO₃⁻, BF₄⁻, AsF₆⁻, PF₆⁻, SbF₃⁻, AlCl₄⁻ , N(SO₂CF₃)₂⁻ und C(SO₂CF₃)₃⁻ besteht .

6. Verwendung der Membran mit erleichtertem Stofftransport gemäss Anspruch 1, wobei das Übergangsmetallsalz ein Komplexsalz des Übergangsmetalls oder eine Mischung von Salzen des Übergangsmetalls umfasst.

7. Verwendung der Membran mit erleichtertem Stofftransport gemäss Anspruch 1, wobei das Polymer gewählt wird aus der Gruppe, die aus Polyethylen, Polypropylen, Polyethylen-co-propylen Kopolymer, Polydimethylsiloxan und deren Kopolymeren und Mischungen besteht.

8. Verwendung der Membran mit erleichtertem Stofftransport gemäss Anspruch 1, wobei die poröse Stützmembran eine poröse Polymermembran oder keramische Membran ist.

9. Verwendung der Membran mit erleichtertem Stofftransport gemäss Anspruch 1, wobei die zu trennenden Kohlenwasserstoffmischungen mindestens einen Alken-Kohlenwasserstoff und/oder mindestens einen Alkan-Kohlenwasserstoff und/oder anderes Gas enthalten.

10. Verwendung der Membran mit erleichtertem Stofftransport gemäss Anspruch 8 oder 9, wobei der Alken-Kohlenwasserstoff gewählt ist aus der Gruppe, die aus Ethylen, Propylen, Butylen, 1,3-Butadien, Isobutylen, Isopren und Mischungen davon besteht, der Alkan-Kohlenwasserstoff gewählt ist aus der Gruppe, die aus Methan, Ethan, Propan, Butan, Isobutan, Pentan-Isomeren und Mischungen davon besteht, und das andere Gas gewählt ist aus der Gruppe, die aus Sauerstoff, Stickstoff, Kohlendioxid, Kohlenmonoxid, Wasser und Mischungen davon besteht.

## Revendications

1. Utilisation d'une membrane de transport facilité comprenant une membrane support poreuse et une membrane de sel d'un métal de transition-polymère consistant en un sel d'un métal de transition et un polymère choisi parmi le groupe consistant en des polyoléfines, des polysiloxanes, ainsi que des copolymères et des mélanges de ceux-ci, dans laquelle le sel d'un métal de transition est dispersé à l'intérieur du polymère qui ne possède pas de groupements fonctionnels capables de former un complexe avec le sel de métal de transition,
pour séparer des hydrocarbures alcènes à partir de mélanges d'hydrocarbures dans des conditions opératoires sèches sans apport de solvants liquides.

2. Utilisation de la membrane de transport facilité selon la revendication 1, dans laquelle un cation d'un sel d'un métal de transition présente une électronégativité de 1,8 à 2,3.

3. Utilisation de la membrane de transport facilité selon la revendication 2, dans laquelle le métal de transition est choisi dans le groupe consistant en Mn, Fe, Co, Ni, Cu, Mo, Tc, Ru, Rh, Pd, Ag, Re, Os, Ir, Pt ainsi que des isomères et des complexes de ceux-ci.

4. Utilisation de la membrane de transport facilité selon la revendication 1, dans laquelle le sel d'un métal de transition possède une énergie réticulaire de moins de 2 500 kJ/mol.

5. Utilisation de la membrane de transport facilité selon la revendication 4, dans laquelle un anion du sel d'un métal de transition est choisi dans le groupe consistant en F⁻, Cl⁻, Br-, I⁻, CN⁻, NO₃⁻, SCN⁻, ClO₄⁻, CF₃SO₃⁻, BP₄⁻, AsF₆⁻, PF⁻₆ , SbF₆⁻, AlCl₄⁻, N(SO₂CF₂)₂⁻ et C(SO₂CF₃)₃⁻.

6. Utilisation de la membrane de transport facilité selon la revendication 1, dans laquelle le sel d'un métal de transition inclut un sel complexe du métal de transition ou un mélange de sels du métal de transition.

7. Utilisation de la membrane de transport facilité selon la revendication 1, dans laquelle le polymère est choisi dans le groupe consistant en un polyéthylène, un polypropylène, un copolymère polyéthylène-co-propylène, un polydiméthyl siloxane, ainsi que des copolymères et des mélanges de ceux-ci.

8. Utilisation de la membrane de transport facilité selon la revendication 1, dans laquelle la membrane support poreuse est une membrane polymère ou une membrane céramique poreuse.

9. Utilisation de la membrane de transport facilité selon la revendication 1, dans laquelle les mélanges d'hydrocarbures à séparer contiennent au moins un hydrocarbure alcène et/ou au moins un hydrocarbure alcane et/ou d'autres gaz.

10. Utilisation de la membrane de transport facilité selon la revendication 8 ou 9, dans laquelle l'hydrocarbure alcène est choisi dans le groupe consistant en l'éthylène, le propylène, le butylène, le 1,3-butadiène, l'isobutylène, l'isoprène et des mélanges de ceux-ci, l'hydrocarbure alcane est choisi dans le groupe consistant en le méthane, l'éthane, le propane, le butane, l'isobutane, le pentane, des isomères et des mélanges de ceux-ci, et un autre gaz est choisi dans le groupe consistant en l'oxygène, l'azote, le dioxyde de carbone, le monoxyde de carbone, l'eau et des mélanges de ceux-ci.
